# EUROPEAN PATENT APPLICATION

(11) **EP 4 394 795 A1**
(43) Date of publication of application: **03.07.2024**
(21) Application number: 23215445.0
(22) Date of filing: 11.12.2023
(51) Int. Cl.: G16H 40/63, G16H 10/60, G16H 15/00, G16H 40/20, G16H 50/30, G16H 50/20, A61B 5/00

(54) **METHOD OF ACQUIRING PHYSIOLOGICAL PARAMETERS OF A PLURALITY OF OBJECTS**

(30) Priority: 29.12.2022 CN 202211716809
(71) Applicant: GE Precision Healthcare LLC, Waukesha, WI 53188 (US)
(72) Inventor: HUI, Hui, Wauwatosa, 53226 (US); JIANG, Menglu, Wauwatosa, 53226 (US); MA, Yu, Wauwatosa, 53226 (US); LI, Dan, Wauwatosa, 53226 (US)
(74) Representative: Kilburn & Strode LLP

(57) **Abstract**

Provided in the present application is a method of acquiring physiological parameters of a plurality of objects, including: generating an information interface about the plurality of objects, the information interface including a personal information tag of each of the plurality of objects; automatically generating a physiological parameter acquisition interface in response to selection of the personal information tag of a current object; and acquiring and evaluating physiological parameters of the current object, and automatically determining a physiological parameter acquisition frequency of the current object on the basis of an evaluation result. Further provided in the present application are a monitor and a non-transitory computer-readable medium.

## Description

### TECHNICAL FIELD

The present invention relates to the field of medical treatment and health, and relates particularly to a method of acquiring physiological parameters of a plurality of objects.

### BACKGROUND

Monitors are conventional medical equipment, and are widely applied to monitoring of vital sign parameters of patents, such as monitoring of parameters including the heart rate, the blood pressure, the respiratory rate, etc., thereby providing medical personnel with timely and accurate feedback about physiological states of the patients.

During actual use, monitors are applied to different scenarios. For example, when medical personnel make ward rounds, the same monitor may be used to acquire physiological parameters of a plurality of objects (e.g., patients). In addition, medical personnel may need to make multiple ward rounds to ensure that changes in physiological parameters of a monitored object can be learned or that a monitored object does not experience serious health risks. Physiological parameter acquisition performed on a plurality of patients during multiple ward rounds involves a great cost in human-hours. In addition, even if ward rounds are made regularly, the risk that physiological parameters of an object are not monitored in a timely manner is still present.

### SUMMARY

The aforementioned defects, deficiencies, and problems are solved herein, and these problems and solutions will be understood through reading and understanding the following description.

Provided in some embodiments of the present application is a method of acquiring physiological parameters of a plurality of objects, comprising: generating an information interface about the plurality of objects, the information interface comprising a personal information tag of each of the plurality of objects; automatically generating a physiological parameter acquisition interface in response to selection of the personal information tag of a current object; and acquiring and evaluating physiological parameters of the current object, and automatically determining a physiological parameter acquisition frequency of the current object on the basis of an evaluation result.

Optionally, the method further comprises: automatically updating the personal information tag of the current object on the basis of the evaluation result, the updating comprising at least one of displaying the evaluation result, displaying the physiological parameter acquisition frequency, and displaying a next acquisition time of the current object.

Optionally, the method further comprises: acquiring and evaluating physiological parameters of each object; and automatically updating the personal information tag of each object on the basis of a physiological parameter evaluation result of each object, comprising: sorting the personal information tags of the respective objects according to a physiological parameter acquisition frequency and/or a next acquisition time of each object.

Optionally, the method further comprises: in response to completion of the acquisition of the physiological parameters of the current object, marking the personal information tag of the current object.

Optionally, the method further comprises: generating a reminder icon on a current interface when the information interface is not opened, the reminder icon being configured to perform at least one of the following: displaying a reminder of a next physiological parameter acquisition in response to being operated; and automatically displaying a physiological parameter acquisition reminder in response to arrival or expiration of a next physiological parameter acquisition time.

Optionally, the method further comprises: when the information interface is not opened, opening the information interface in response to a sliding touch from a side edge of a current interface.

Optionally, generating an information interface of the plurality of objects comprises at least one of the following:
carrying out the generation by reading a storage medium storing personal information of the plurality of objects; carrying out the generation in response to personal information of the plurality of objects being input via an input apparatus; and carrying out the generation in response to personal information of the plurality of objects being imported via an electronic medical record system.

Optionally, automatically determining a physiological parameter acquisition frequency of the current object on the basis of an evaluation result comprises: generating an early warning score on the basis of the physiological parameters of the current object, and automatically determining the physiological parameter acquisition frequency of the current object on the basis of the early warning score.

Optionally, automatically determining a physiological parameter acquisition frequency of the current object on the basis of an evaluation result comprises: predefining a scoring criterion for each physiological parameter; comparing each physiological parameter of the current object with the scoring criterion to determine a score for each physiological parameter; and determining a resulting total score of the current object on the basis of the score of each physiological parameter, so as to automatically determine the physiological parameter acquisition frequency of the current object.

Further provided in some embodiments of the present application is a monitor, comprising: a touch screen, configured to display information, and to generate an input signal in response to being touched; a processor, configured to perform the method according to any one of the foregoing; and a plurality of physiological parameter acquisition devices, configured to acquire physiological parameters.

Further provided in some embodiments of the present application is a non-transitory computer-readable medium, having a computer program stored therein, the computer program having at least one code segment, and the at least one code segment being executable by a machine to perform the steps of the method according to any one of the foregoing.

It should be understood that the brief description above is provided to introduce, in a simplified form, concepts that will be further described in the detailed description. The brief description above is not meant to identify key or essential features of the claimed subject matter. The scope is defined uniquely by the claims that follow the detailed description. Furthermore, the claimed subject matter is not limited to implementations that solve any deficiencies raised above or in any section of the present disclosure.

### BRIEF DESCRIPTION OF THE DRAWINGS

The present application will be better understood by reading the following description of non-limiting embodiments with reference to the accompanying drawings, wherein:
FIG. 1 is a schematic diagram of a monitor according to some embodiments of the present application;
FIG. 2 is a flowchart of acquiring physiological parameters of a plurality of objects according to some embodiments of the present application;
FIG. 3 is an information interface including personal information tags of a plurality of objects in some embodiments of the present application;
FIG. 4 is a schematic diagram of a physiological parameter acquisition interface in some embodiments of the present application;
FIG. 5 is a schematic diagram of an information interface after acquisition performed on some objects is completed according to some embodiments of the present application;
FIG. 6 is a schematic diagram of an information interface in some other embodiments of the present application;
FIG. 7 is a schematic diagram of an interface where a reminder icon is operated to acquire a reminder of a next physiological parameter acquisition in some embodiments of the present application; and
FIG. 8 is a schematic diagram of an interface where a reminder icon proactively generates a physiological parameter acquisition reminder in some embodiments of the present application.

### DETAILED DESCRIPTION

Specific implementations of the present invention will be described in the following. It should be noted that in the specific description of the implementations, it is impossible to describe all features of the actual implementations of the present invention in detail, for the sake of brief description. It should be understood that in the actual implementation process of any embodiment, just as in the process of any one engineering project or design project, a variety of specific decisions are often made to achieve specific goals of the developer and to meet system-related or business-related constraints, which may also vary from one embodiment to another. Furthermore, it should also be understood that although efforts made in such development processes may be complex and extended, for a person of ordinary skill in the art related to the disclosure of the present invention, some design, manufacture, or production changes made on the basis of the technical disclosure of the present disclosure are only common technical means, and should not be construed as the content of the present disclosure being insufficient.

Unless otherwise defined, the technical or scientific terms used in the claims and the description should have the usual meanings understood by those of ordinary skill in the technical field to which the present invention belongs. "First", "second" and similar words used in the present invention and the claims do not denote any order, quantity or importance, but are merely intended to distinguish between different constituents. The terms "one" or "a/an" and similar terms do not express a limitation of quantity, but rather that at least one is present. The terms "include" or "comprise" and similar words indicate that an element or object preceding the terms "include" or "comprise" encompasses elements or objects and equivalent elements thereof listed after the terms "include" or "comprise", and do not exclude other elements or objects. The terms "connect" or "link" and similar words are not limited to physical or mechanical connections, and are not limited to direct or indirect connections.

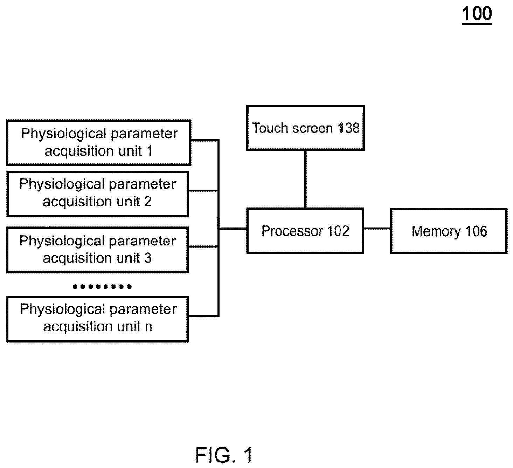

Monitors are medical equipment widely used in clinical practice. FIG. 1 shows a schematic diagram of a monitor 100 according to some embodiments of the present application. As shown in FIG. 1, the monitor 100 may include a plurality of physiological parameter acquisition units 1 to n, a processor 102, a touch screen 138, and a memory 106.

The physiological parameter acquisition units 1 to n may be different types of sensors. In some examples, these different types of sensors may be used to acquire different physiological parameters, including, but not limited to, non-invasive blood pressure, body temperature, heart rate, respiratory rate, blood oxygen saturation, etc. Specific structures and acquisition principles of the sensors may be arbitrary in the art, and are not described herein. The physiological parameter acquisition units 1 to n can be electrically connected to the processor 102 so as to acquire physiological parameters under the control of the processor 102, and the acquired physiological parameters can be sent to the processor 102 to be processed.

The processor 102 may include one or more processors. For example, the processor 102 may include a central processing unit (CPU), one or more microprocessors, or any other electronic assembly capable of processing input data according to specific logic instructions. The processor 102 may include one or more hardware circuits (not shown). The processor 102 is connected to the physiological parameter acquisition units by means of the hardware circuits, so as to control acquisition of physiological parameters, receive the acquired physiological parameters, and process the same.

The processor 102 is electrically connected to the touch screen 138. The touch screen 138 is configured to display information, and to generate an input signal in response to being touched. The displayed information may be information received from the processor 102. For example, the displayed information may be a physiological parameter acquisition result, a monitor configuration page, an object management page, etc. The input signal may be generated due to the user's touch, such as the touch of a finger. The touch screen 138 has functions of both a display apparatus and an input apparatus. In one example, the touch screen 138 may be a capacitive touch screen. In response to the touch of the user's finger, a change in capacitance may occur in a touched position on the touch screen 138 to generate an electrical signal. In another example, the touch screen 138 may be a resistive touch screen, and a change in resistance may occur in response to the touch of the user's finger. Examples will not be further enumerated herein.

The processor 102 is further electrically connected to the memory 106. The memory 106 may be used to store parameters, algorithms, etc., used by the processor 102 to perform one or more operations described in the present application. In addition, the memory 106 may also store acquired physiological parameters. The memory 106 may be a tangible and non-transitory computer-readable medium such as a flash memory, a RAM, a ROM, an EEPROM, etc.

In one example, the monitor 100 is configured to be fixed in a desired location of use, e.g., on a wall of a ward. In another example, the monitor 100 is configured to be portable, and is provided with a dedicated portable movable cart. In addition, it can be understood that the monitor 100 according to any embodiment of the present application may further include, in addition to the components described above, other components, such as a power source, a switch, a button, etc. Examples will not further enumerated herein.

In a use scenario, the monitor 100 aids a user in making a ward round. During the ward round, physiological parameters of a plurality of objects need to be acquired one by one, and a plurality of rounds need to be performed within a certain time interval. Frequently making ward rounds imposes greater physical labor on medical personnel. However, a reduction in the frequency of making ward rounds may incur the risk of overlooking deteriorating conditions of critical patients. Considering at least the problems described above, the inventor provides an improved method of acquiring physiological parameters.

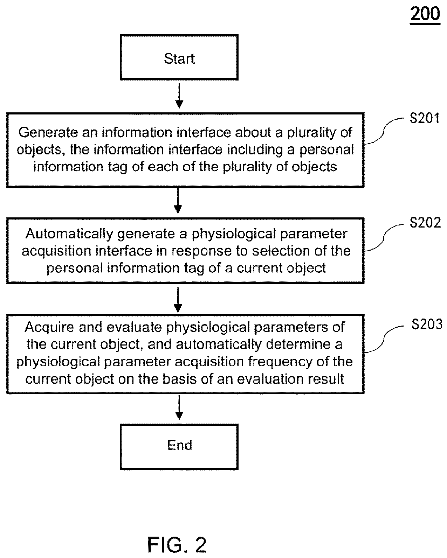

Referring to FIG. 2, FIG. 2 shows a flowchart of acquiring physiological parameters of a plurality of objects in some embodiments of the present application.

In step 201, an information interface about the plurality of objects is generated, and the information interface includes a personal information tag of each of the plurality of objects.

The process may be implemented by a processor, e.g., the processor of the monitor 100 depicted in FIG. 1 and described in related embodiments thereof. The processor may control a touch screen to generate an information interface dedicated to ward rounds, and the information interface includes information about a plurality of objects (e.g., patients on a plurality of ward beds) needing to undergo physiological parameter acquisition. In addition, the information about the plurality of objects is configured to be presented independently, that is, configured to include a personal information tag of each object. This configuration enables the user to easily learn information about each object on which acquisition needs to be performed, and facilitate operation of the next step. The centralized information interface eliminates the defect that a conventional monitor needs to manage information one by one when acquiring physiological information of different patients, thereby greatly improving acquisition efficiency.

In step 202, a physiological parameter acquisition interface is automatically generated in response to selection of the personal information tag of a current object.

The process may be implemented by a processor. When one round of acquisition of physiological parameters of the plurality of objects needs to be performed, one of the plurality of objects may be selected as a current object on which acquisition needs to be performed. For example, the user may use a finger to tap the personal information tag of the current object on the information interface. Upon receiving a signal indicating that the personal information tag is selected, the processor may automatically generate the physiological parameter acquisition interface to allow the user to perform acquisition easily. The physiological parameter acquisition interface may be automatically generated in a variety of manners. In one example, upon receiving a signal indicating that the personal information tag is tapped, the processor directly determines that the tag is selected, and therefore automatically generates and displays the physiological parameter acquisition interface, thereby reducing work procedures. In another example, upon receiving a signal indicating that the personal information tag is tapped, the processor may generate a confirmation window to allow the user to reconfirm the selection, and then automatically generate and display the physiological parameter acquisition interface after the user has reconfirmed the selection, thereby reducing the possibility of incorrect operations. Examples will not be further enumerated herein.

In step 203, physiological parameters of the current object are acquired and evaluated, and a physiological parameter acquisition frequency of the current object is automatically determined on the basis of an evaluation result.

The process may be implemented by a processor. The user may connect the physiological parameter acquisition devices to the current object in an appropriate manner. In this case, the processor can receive physiological parameters of the current object acquired by the physiological parameter acquisition devices. Moreover, the processor is further configured to evaluate the acquired physiological parameters, determine a physiological condition of the current object by means of evaluation of the physiological parameters, and further automatically determine which physiological parameter acquisition frequency should be used to perform multiple rounds of physiological parameter acquisition for the current object. In one example, when the processor determines that the physiological parameters of the current object are within the normal range, the processor may automatically determine that physiological parameter acquisition is to be performed on the current object at an appropriate lower frequency. In another example, when the processor determines that the physiological parameters of the current object are not within the normal range, the processor may automatically determine that physiological parameter acquisition is to be performed on the current object at an appropriate high frequency.

In the embodiments described above, in one aspect, the information about the objects requiring acquisition is displayed on the information interface in a centralized manner, thereby improving user efficiency in performing information management and parameter acquisition on the objects to be subjected to acquisition. In another aspect, the processor can automatically determine different acquisition frequencies for different objects according to acquisition results, thereby preventing objects in good conditions from being subjected to unnecessary repeated acquisition, and ensuring that acquisition performed on objects in poor conditions is not missed.

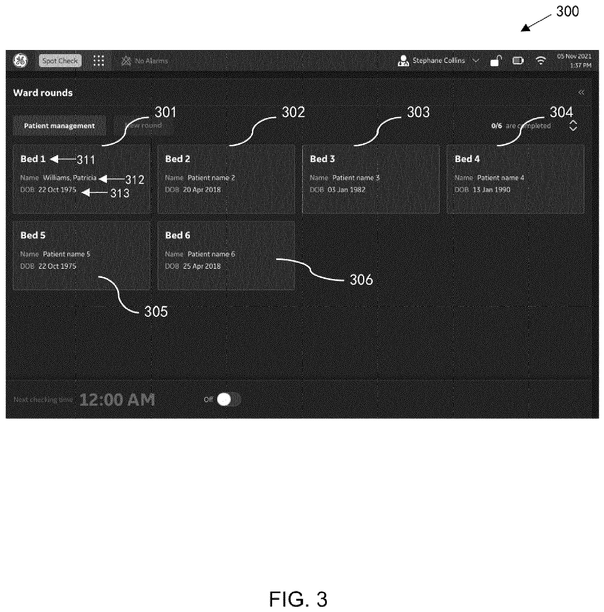

In order to more clearly describe embodiments of the present application, FIG. 3 to FIG. 8 exemplarily show a user interface of the present application.

Referring first to FIG. 3, FIG. 3 shows an information interface 300 including personal information tags of a plurality of objects in some embodiments of the present application. The information interface 300 includes a plurality of personal information tags 301 to 307. In one example, these personal information tags are arranged according to a certain sequence, for example, from top to bottom and from left to right, thereby making it easier for the user to manage patient information. In addition, the personal information tag includes basic information required for a ward round. Description is provided with reference to the personal information tag 301. The personal information tag 301 includes information such as a bed number 311, the name 312 of a current object, the date of birth 313 of the current object, etc. The information can ensure that medical personnel accurately find a desired bed when making a ward round, and determine whether an object is correct. In addition, it can be understood that the personal information tag may further include other types of information, e.g., information that makes it easier for the medical personnel to make a decision, such as medical record information, special notes, etc., about the current object. In addition, the personal information tag in FIG. 3 is configured to be in the form of a rectangular card. In light of the teaching of the present application, the personal information tag may also be of other types.

In addition, it should be understood that the information interface of the plurality of objects described above may be generated in a variety of manners. An exemplary description is provided below.

In one example, the information interface may be generated by the processor according to personal information of the plurality of objects input via an input apparatus. For example, the user may input the personal information of the plurality of objects in a centralized manner by operating the touch screen. Furthermore, in addition to the touch screen, the input apparatus may also be in other forms, such as a microphone for voice input, an external keyboard, a mouse, etc.

In another example, the information interface may be generated by the processor according to a storage medium that is read by the processor and that stores personal information of the plurality of objects described above. For example, the user may use an apparatus such as a barcode scanner or the like to read barcode wristbands including the personal information of the respective objects one by one, and sends the same to the processor via the barcode scanner, so that the processor generates the information interface. Alternatively, the information interface may be automatically generated by the processor by reading a storage medium storing the personal information of the objects, such as an IC card, a USB flash drive, or the like.

In other examples, the information interface may also be automatically generated by the processor according to received personal information of objects imported by an electronic medical record system (EMR). The generation of the information interface of the plurality of objects in combination with the electronic medical record system can greatly reduce efforts required from the user, thereby improving working efficiency.

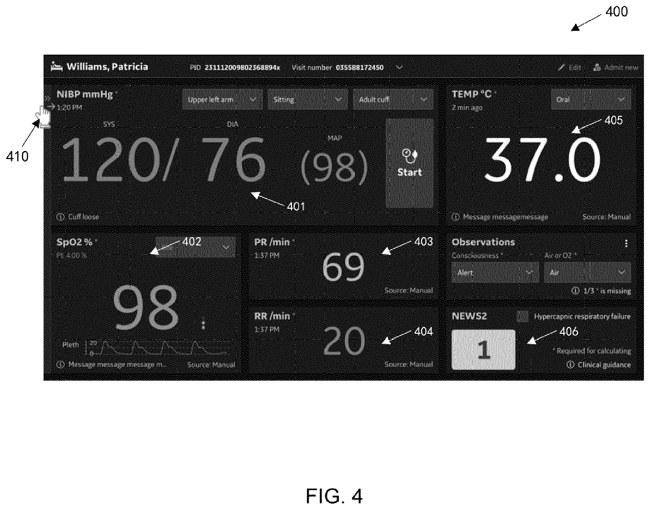

Further, referring to FIG. 4, FIG. 4 shows a schematic diagram of a physiological parameter acquisition interface 400 in some embodiments of the present application. When the user prepares to acquire physiological parameters of the plurality of objects, the user may select the personal information tag of any object on the information interface, so as to acquire the physiological parameters of the current object. By means of selection, the information interface may jump to the physiological parameter acquisition interface 400 shown in FIG. 4. In this case, the physiological parameters can be acquired as soon as the physiological parameter acquisition devices are connected to the current object. The acquired physiological parameters can be displayed on the acquisition interface in real time. The physiological parameters, for example, may include non-invasive blood pressure 401, blood oxygen saturation 402, heart rate 403, respiratory rate 404, body temperature 405, etc. In this case, the monitor for ward rounds can be substantially understood as having common monitor functions, and can perform continuous monitoring on the physiological parameters of the current object. In addition, the current physiological parameter acquisition interface may further include an early warning score 406. The early warning score may be any scoring system used in the art, such as EWS, NEWS2, MEWS, or the like. The early warning score is acquired according to the physiological parameters of the current object. The scores of the physiological parameters are respectively acquired according to differences between the physiological parameters and reference values, and are added to acquire the sum, so as to ultimately acquire the early warning score. The early warning score can reflect the degree of risk that the current object is at.

As described above in the present invention, in the embodiments of the present application, the physiological parameters of the current object may also be evaluated, and the physiological parameter acquisition frequency of the current object is automatically determined on the basis of an evaluation result. In one example, the automatic determination described above may be that the physiological parameter acquisition frequency of the current object is automatically determined on the basis of the early warning score 406. As shown in FIG. 4, if the early warning score 406 of the current object has been determined, the processor may determine, according to the value of the warning score, the frequency at which the physiological parameters of the current object need to be acquired, that is, when to perform the next round of acquisition.

It can be understood that the automatic determination described above may also be performed in other manners. In another example, the user may be granted a higher degree of freedom. For example, a scoring criterion for each physiological parameter may be predefined. Further, each physiological parameter of the current object is compared with the scoring criterion to determine a score for each physiological parameter. Finally, a resulting total score of the current object is determined on the basis of the scores of the respective physiological parameters, thereby automatically determining the physiological parameter acquisition frequency of the current object. The predefinition may be performed by the user according to different clinical requirements. For example, the scoring criteria for the physiological parameters of the objects may be adjusted according to different situations of the objects. For example, for an object having a severe arrhythmia, the heart rate thereof may require special attention, and in this case, the scoring criterion for the heart rate thereof may be stricter compared to an object receiving normal attention, and is assigned a greater weight. Alternatively, the predefined criterion may also vary from department to department. Different departments may focus on different indicators, and different scoring criteria may be configured accordingly. In addition, the predefinition may be stored so as to be used in the next physiological parameter acquisition. After the predefined scoring criterion is employed, each physiological parameter of the current object may be scored, and a total score is calculated. Then, the physiological parameter acquisition frequency is automatically determined according to the total score. For a patient whose score structure indicates a high degree of risk, a high acquisition frequency is assigned. For a patient whose score result indicates a low degree of risk, a low acquisition frequency is assigned. In this manner, both safety and checking efficiency are considered.

After the acquisition of the physiological parameters of the current object is completed, the process described above may be repeated to continue to acquire the physiological parameters of a next object until acquisition on all objects requiring physiological parameter acquisition is completed. The objects requiring physiological parameter acquisition may be all the objects on the information interface, or may be objects selected by the user to perform acquisition according to actual situations. Switching from acquisition performed on a current object to acquisition performed on the next object is exemplary described below.

First, with continued reference to FIG. 4, after the acquisition of the physiological parameters of the current object is completed, the user interface may be caused to jump from the current physiological parameter acquisition interface to the information interface. The jump may be caused via an operation performed by the user. For example, when the information interface is not opened, the processor may open the information interface in response to a sliding touch from a side edge 410 of a current interface. As shown in FIG. 4, the side edge 410 of the current interface is configured to be operable. When the user touches and slides the side edge 410, the information interface is opened, and in this case, the physiological parameter acquisition interface is closed. It should be noted that the interface switching described above may also be switching from another interface to the information interface, for example, switching from a monitor system setting interface or another interface.

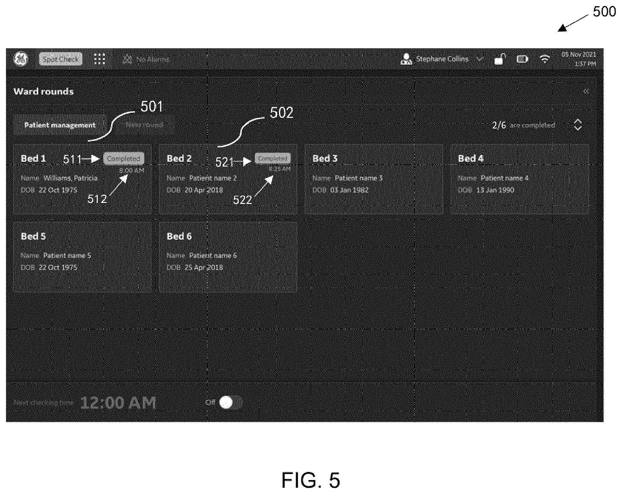

Referring to FIG. 5, FIG. 5 shows a schematic diagram of an information interface 500 after acquisition performed on some objects is completed according to some embodiments of the present application. In this case, the user may operate the information interface 500 to continue to acquire the physiological parameters of the next object. A specific execution means may be similar to that in the embodiments described above in the present application, and will not be described herein again.

Further, considering that when too many personal information tags are present on the information interface, the user may be confused as to which objects have already undergone acquisition, improvements are made in some embodiments of the present application. Specifically, the processor marks the personal information tag of the current object in response to completion of the acquisition of the physiological parameters of the current object. Referring to FIG. 5, the acquisition performed on current objects is completed, and in this case, the personal information tags 501, 502 thereof can be marked. The marking method may include at least one of an acquisition completion mark 511, 521 and an acquisition time mark 512, 522. This configuration can prevent the user from missing an acquisition or repeating the same acquisition, thereby improving acquisition efficiency.

As described in the embodiments of the present application described above, after the acquisition of the physiological parameters of an object is completed, the acquisition frequency thereof is automatically determined by the processor. Considering that visualization of the above frequency facilitates decision-making by the user, the personal information tag on the information interface is further optimized in some embodiments of the present application. In some embodiments, the processor may automatically update the personal information tag of the current object on the basis of an evaluation result.

In one example, the updating includes displaying the evaluation result. For example, when the early warning score serves as the evaluation, the early warning score of the current object that has completed the physiological parameter acquisition may be displayed on the personal information tag corresponding thereto.

In one example, the updating includes displaying the physiological parameter acquisition frequency. For example, when the processor determines, according to the evaluation result, that the physiological parameter acquisition frequency of the current object is 2 hours/time, the personal information tag can be updated with said frequency (2 times/h).

In one example, the updating includes displaying a next acquisition time of the current object. For example, when the processor determines, according to the evaluation result, that the physiological parameter acquisition frequency of the current object is 2 hours/time, the processor may be configured to automatically add an acquisition time interval and a current acquisition completion time to obtain the sum so as to acquire the next acquisition time, and the personal information tag of the current object is then updated so that the next acquisition time is displayed thereon.

In one example, the updating may be at least one of displaying the evaluation result, displaying the physiological parameter acquisition frequency, and displaying a next acquisition time of the current object described above.

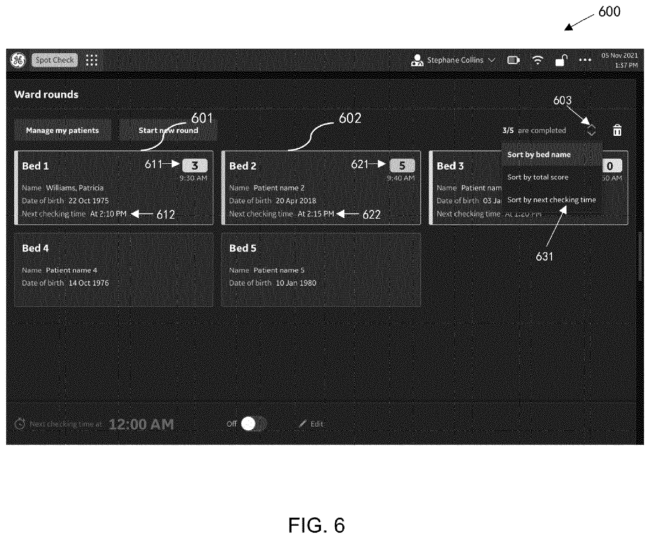

A graphical description of the updating of the personal information tag is provided below. Referring to FIG. 6, FIG. 6 shows a schematic diagram of an information interface in some other embodiments of the present application. As shown in FIG. 6, after the user acquires the physiological parameters of some objects, the personal information tags of the objects that have already undergone the acquisition are updated. In the information interface shown in FIG. 6, a personal information tag 601 and a personal information tag 602 additionally include evaluation results, i.e., early warning scores 611, 621 and next checking times 621, 622, as compared to the time before the acquisition. This configuration enables the user to clearly know which objects need to undergo acquisition more frequently and which objects may undergo acquisition at an appropriate lower frequency when the user needs to browse all objects needing to undergo multiple rounds of physiological parameter acquisition.

It can be understood that the embodiments described above describe acquiring and evaluating the physiological parameters of some objects. Similarly, the physiological parameters of each object may be acquired and evaluated, and the personal information tag of each object is automatically updated on the basis of a physiological parameter evaluation result of the object. In addition to the updating described above, the updating may further include: sorting the personal information tags of the respective objects according to a physiological parameter acquisition frequency and/or a next acquisition time of each object. In one example, the sorting described above may be performed by the processor after the physiological parameter acquisition frequency of each object is determined. In another example, the sorting described above may be performed by the user after inputting an operating instruction. With continued reference to FIG. 6, an exemplary description is provided. In the embodiment shown in FIG. 6, the information interface includes a sorting option 603 for selection by the user. The user can select different sorting types by selecting (e.g., tapping) the sorting option 603. When a sorting type 631 "sort by next checking time" is selected, the personal information tags of objects having earlier acquisition times are arranged up front. This configuration makes it easier for the user to increase the acquisition frequency.

It is recognized by the inventor that during actual use, the user needs to switch frequently between the information interface and the physiological parameter acquisition interface, and the user interface needs to stay in the physiological parameter acquisition interface for a long time. In this case, the risk of missing a next physiological parameter acquisition time is present. At least in view of this, improvements are made in some embodiments of the present application. Specifically, the processor may be configured to generate a reminder icon on a current interface when the information interface is not opened. The reminder icon is configured to perform at least one of the following: displaying a reminder of a next physiological parameter acquisition in response to being operated; and automatically displaying a physiological parameter acquisition reminder in response to the arrival of a next physiological parameter acquisition time. The reminder icon ensures that normal functions of the current interface are not affected. In addition, when a reminder needs to be provided in the case of an emergency, the reminder icon also has the function of reminding the operator. It can be understood that the next physiological parameter acquisition described above may be performed on the current object, or may be performed on any other object included in the information interface.

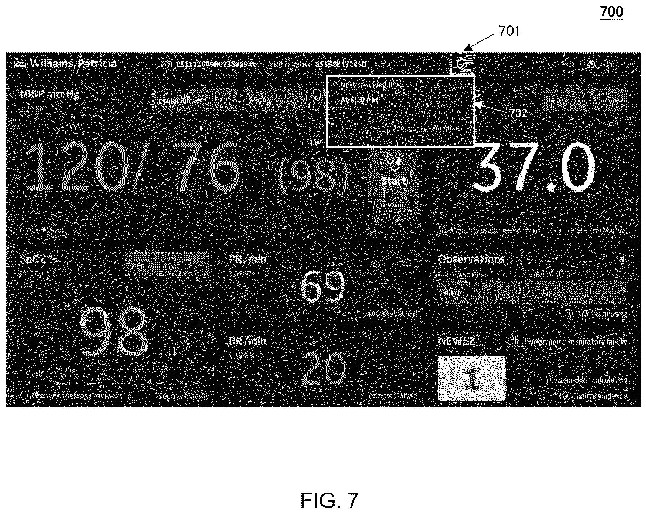

In one example, before the arrival of the next physiological parameter acquisition, the reminder icon does not need to proactively provide a reminder function. In this case, the processor turns on and displays a reminder of the next physiological parameter acquisition only when the user operates, for example taps on, the reminder icon. This configuration enables the user to quickly learn the next physiological parameter acquisition time without entering the information interface unnecessarily, and do not affect functions of the current interface. Referring to FIG. 7, FIG. 7 is a schematic diagram of an interface 700 where a reminder icon is operated to acquire a reminder of a next physiological parameter acquisition in some embodiments of the present application. A reminder icon 701 is arranged at an appropriate position on the interface 700, such as the top, thereby preventing the current interface from being affected. A reminder interface 702 is displayed only when the user taps on the reminder icon 701, and the information displayed includes a next physiological parameter acquisition time. The user can quickly learn the next acquisition time without switching the interface, so that continuity of operation on the current interface is not interrupted. It can be understood that FIG. 7 shows one example, and the position, size, color, etc., of the reminder icon can be adjusted in light of the teaching of the present application.

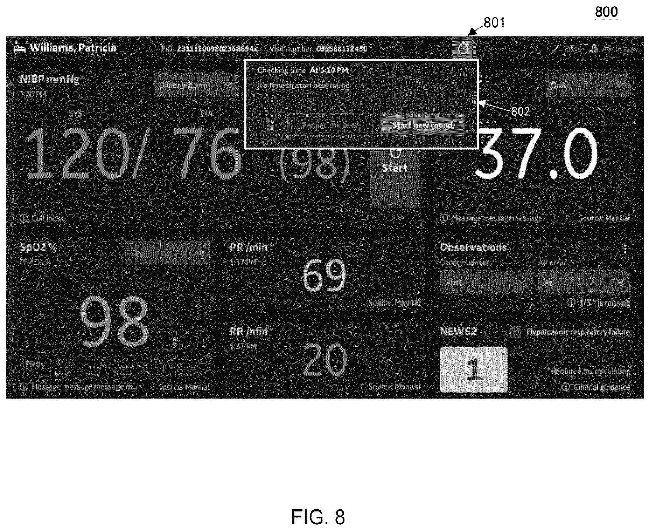

In another example, upon arrival or expiration of the next physiological parameter acquisition time, the reminder icon automatically displays a physiological parameter acquisition reminder so as to more expressly remind the user to perform the next physiological parameter acquisition. In this way, a risk resulting from the user forgetting to perform acquisition is avoided. Referring to FIG. 8, FIG. 8 shows a schematic diagram of an interface 800 where a reminder icon proactively generates a physiological parameter acquisition reminder in some embodiments of the present application. The position and the display method of a reminder icon 801 may be the same as those shown in FIG. 7, and will not be described again. In FIG. 8, a next physiological parameter acquisition time has arrived, and in this case, the processor can control the reminder icon to proactively display a physiological parameter acquisition reminder 802 to remind the user. It can be understood that, if the next physiological parameter acquisition time has expired, the processor may control the reminder icon to proactively display the physiological parameter acquisition reminder in a similar manner or in response to a selection operation performed by the user (not shown in the drawing), and details will not be described again.

It will be appreciated that the above merely schematically illustrates the embodiments of the present application, but the present application is not limited thereto. For example, the order of execution between operations may be appropriately adjusted. In addition, some other operations may be added or some operations may be omitted. A person skilled in the art could make appropriate variations according to the above disclosure, rather than being limited by the above descriptions.

Further provided in some embodiments of the present application is a monitor, including: a touch screen, configured to display information, and to generate an input signal in response to being touched; a processor, configured to perform the method according to any one of the embodiments described above; and a plurality of physiological parameter acquisition devices, configured to acquire physiological parameters.

Further provided in some embodiments of the present application is a non-transitory computer-readable medium, having a computer program stored therein, the computer program having at least one code segment, and the at least one code segment being executable by a machine so as to enable the machine to perform the steps of the method in any of the embodiments described above.

Correspondingly, the present disclosure may be implemented as hardware, software, or a combination of hardware and software. The present disclosure may be implemented in at least one computer system in a centralized manner or in a distributed manner, different elements in the distributed manner being distributed on a number of interconnected computer systems. Any type of computer system or other device suitable for implementing the methods described herein is considered to be appropriate.

The various embodiments may also be embedded in a computer program product, which includes all features capable of implementing the methods described herein, and the computer program product is capable of executing these methods when loaded into a computer system. The computer program in this context means any expression in any language, code, or symbol of an instruction set intended to enable a system having information processing capabilities to execute a specific function directly or after any or both of a) conversion into another language, code, or symbol; and b) duplication in a different material form.

The purpose of providing the above specific embodiments is to facilitate understanding of the disclosure of the present invention more thoroughly and comprehensively, but the present invention is not limited to these specific embodiments. Those skilled in the art should understand that various modifications, equivalent replacements, and changes can also be made to the present invention and should be included in the scope of protection of the present invention as long as these changes do not depart from the spirit of the present invention.

## Claims

1. A method of acquiring physiological parameters of a plurality of objects, comprising:
generating an information interface about the plurality of objects, the information interface comprising a personal information tag of each of the plurality of objects;
automatically generating a physiological parameter acquisition interface in response to selection of the personal information tag of a current object; and
acquiring and evaluating physiological parameters of the current object, and automatically determining a physiological parameter acquisition frequency of the current object on the basis of an evaluation result.

2. The method according to claim 1, further comprising:
automatically updating the personal information tag of the current object on the basis of the evaluation result, the updating comprising at least one of displaying the evaluation result, displaying the physiological parameter acquisition frequency, and displaying a next acquisition time of the current object.

3. The method according to claim 1, further comprising:
acquiring and evaluating physiological parameters of each object; and
automatically updating the personal information tag of each object on the basis of a physiological parameter evaluation result of each object, comprising: sorting the personal information tags of the respective objects according to a physiological parameter acquisition frequency and/or a next acquisition time of each object.

4. The method according to claim 1, further comprising:
in response to completion of the acquisition of the physiological parameters of the current object, marking the personal information tag of the current object.

5. The method according to claim 1, further comprising:
generating a reminder icon on a current interface when the information interface is not opened, the reminder icon being configured to perform at least one of the following:
displaying a reminder of a next physiological parameter acquisition in response to being operated; and
automatically displaying a physiological parameter acquisition reminder in response to arrival or expiration of a next physiological parameter acquisition time.

6. The method according to claim 1, further comprising:
when the information interface is not opened, opening the information interface in response to a sliding touch from a side edge of a current interface.

7. The method according to claim 1, wherein generating an information interface of the plurality of objects comprises at least one of the following:
carrying out the generation by reading a storage medium storing personal information of the plurality of objects;
carrying out the generation in response to personal information of the plurality of objects being input via an input apparatus; and
carrying out the generation in response to personal information of the plurality of objects being imported via an electronic medical record system.

8. The method according to claim 1, wherein automatically determining a physiological parameter acquisition frequency of the current object on the basis of an evaluation result comprises:
generating an early warning score on the basis of the physiological parameters of the current object, and automatically determining the physiological parameter acquisition frequency of the current object on the basis of the early warning score.

9. The method according to claim 1, wherein automatically determining a physiological parameter acquisition frequency of the current object on the basis of an evaluation result comprises:
predefining a scoring criterion for each physiological parameter;
comparing each physiological parameter of the current object with the scoring criterion to determine a score for each physiological parameter; and
determining a resulting total score of the current object on the basis of the score of each physiological parameter, so as to automatically determine the physiological parameter acquisition frequency of the current object.

10. A monitor, comprising:
a touch screen, configured to display information, and to generate an input signal in response to being touched;
a processor, configured to perform the method according to any one of claims 1 to 9; and
a plurality of physiological parameter acquisition devices, configured to acquire physiological parameters.

11. A non-transitory computer-readable medium, having a computer program stored therein, the computer program having at least one code segment, and the at least one code segment being executable by a machine to perform the steps of the method according to any one of claims 1 to 9.
